# EUROPEAN PATENT SPECIFICATION

(11) **EP 3 115 914 B1**
(45) Date of publication and mention of the grant of the patent: **17.01.2018**
(21) Application number: 16179841.8
(22) Date of filing: 22.02.2011
(51) Int. Cl.: G06F 17/40, A61B 5/00, A61B 5/022

(54) **BODY PARAMETER SENSOR AND MONITOR INTERFACE**
KÖRPERPARAMETERSENSOR UND MONITORSCHNITTSTELLE
CAPTEUR DE PARAMÈTRE CORPOREL ET INTERFACE DE MONITEUR

(30) Priority: 24.02.2010 US 711425
(43) Date of publication of application: 11.01.2017
(62) Divisional of application: 11747930.3
(73) Proprietor: Edwards Lifesciences Corporation, Irvine, CA 92614 (US)
(72) Inventor: Phan, Luong Ngoc, Irvine, CA 92614 (US); Backer, Steven, Irvine, CA 92614 (US); Wilbur, Jennifer, Irvine, CA 92614 (US); Higgins, Michael J., Irvine, CA 92614 (US)
(74) Representative: Eisenführ Speiser

(56) References cited:
- WO-A1-2007/117405
- WO-A2-2007/014527
- WO-A2-2008/054976
- WO-A2-2009/127954

## Description

### FIELD OF THE INVENTION

The present invention relates generally to sensors of physiological parameters, such as pressure transducers and fluid monitors, and, more particularly, to disposable physiological sensors and associated patient monitor interfaces.

### BACKGROUND OF THE INVENTION

When diagnosing and treating various bodily ailments, such as with patients suffering from shock or cardiovascular problems, medical personnel often find it desirable to measure or monitor a patient's blood pressure and/or other physiological parameters. Advantageously, by measuring or monitoring physiological parameters of these and other types of patients, medical personnel are better able to detect medical difficulties and other problems at an early stage. As a result, the use of physiological sensors and associated monitoring may increase the likelihood that a patient can be successfully treated or provided with needed emergency assistance.

A variety of methods are currently used for measuring or monitoring blood pressure. For example, medical personnel frequently use various indirect blood pressure measurement techniques, such as measuring a patient's blood pressure with a pressure cuff and a stethoscope. In addition, blood pressure measurements can also be made using a number of direct measurement and monitoring techniques, such as use of a disposable pressure transducer (DPT) or with other disposable medical devices (such as a catheter) that have an integrated/embedded DPT. Notably, when diagnosing or treating critically ill patients, such direct techniques are usually preferred over any of the indirect techniques. Direct blood pressure measurement and monitoring techniques are generally accurate to within about one percent and facilitate the continuous monitoring of a patient's blood pressure on a beat-to-beat basis. Direct blood pressure monitoring also enables the rapid detection of a change in cardiovascular activity, and this may be of significant importance in emergency situations.

For direct, or invasive, blood pressure monitoring systems a catheter is inserted into a patient's circulatory system with the end of the catheter having an opening to the blood stream, typically in a major or peripheral blood vessel. First, a needle is inserted into a peripheral blood vessel. For example, if it is desired to monitor arterial blood pressure, the needle may be inserted into the radial artery. If, on the other hand, venous blood pressure is to be monitored, the needle may be inserted into the antecubital, radial, jugular, or subclavian veins. Once the needle is properly inserted, a special catheter is threaded through the needle and into the blood vessel until the tip of the catheter is positioned at the particular point within the body at which it is desired to make the blood pressure measurement. Then, with the catheter in place, the needle may be withdrawn.

An I.V. set attaches to the proximal end of the catheter protruding from the patient so that a solution flows through the catheter and into the patient. The I.V. solution provides a fluid "column" through which pressure pulses are transmitted, and a pressure transducer positioned along the fluid column monitors those pressure pulses. Generally, the pressure transducer consists of a dome that functions as a reservoir for the I.V. fluid. The dome includes a resilient diaphragm that attaches to an electrical transducer. The transducer senses pressure fluctuations in the diaphragm and converts them into electrical signals which are then transmitted through a cable to a monitor for amplification and display. In modern systems a single silicon chip comprises both the pressure diaphragm and the measuring circuitry of the pressure transducer. The cable includes a connector so that the transducer and associated portion of the cable can be discarded after use, whereas the mating connector and cable hard-wired to the monitor can be reused. Such disposable blood pressure transducers (DPTs) are the standard of care in the OR, ICU or CCU.

Due to the separable nature of the transducer and monitor, different transducers may be connected to any one monitor, as long as the cable connectors are compatible. However, transducers from different sources may exhibit different performance characteristics and may require specific calibration or signal processing or conditioning. Unfortunately, the environments of the OR, ICU or CCU are ill-suited for rapid recognition and registration of disparate components of pressure monitoring systems, and safety concerns necessitate the least amount of such preparation be involved.

Furthermore, pressure data are often required by two separate monitoring devices, such as a patient monitor and a cardiac output monitor, or a patient monitor and an aortic balloon pump. Typically, an arterial line is placed in the patient and a DPT connected to a patient monitor is used for pressure monitoring. Instead of invasively setting up a second arterial line and DPT, the signal from the first DPT may be supplied to a second monitor via the patient monitor (or via the cabling by splitting the signal). However, this "piggyback" connection may introduce pressure monitoring errors from delays and distortion of the signals.

Movement of a patient from one location to another also can present problems. The monitor is usually immobile and stays behind when the patient is moved. Removing and reinserting a sensor in the patient is generally undesirable, therefore the sensor usually remains in the patient and is then plugged into a new monitor at the new location. Data continuity is disrupted, therefore, each time a patient is moved.

Despite a relatively mature market for disposable medical pressure transducers, there remains a need for an improved transducer (and other body parameter sensor systems) that when interfaced with the appropriate monitoring device ensures accuracy and continuity of sensor data. There is also a need for a system that authenticates the sensor and/or for storing data in memory local to the sensor that can be transported with the sensor from location-to-location. Other sensor systems, e.g. as known from WO-2007/117405, suffer similar defects.

### SUMMARY OF ASPECTS OF THE INVENTION

One aspect of the invention is a sensor system for sensing a physiological parameter in a human or animal. The system includes a physiological sensor, and memory and a microprocessor local to and fixedly attached to the sensor. The sensor can be any of a variety of sensors that output a sensor signal representative of a sensed physiological parameter. An authentication algorithm is stored in the memory that is local to the sensor, and the system is configured to engage in an authentication process to authenticate the sensor when queried by a remote processor. In one embodiment, the memory is configured to receive and store data from a remote processor, which may be as one example data that is representative of a physiological parameter sensed by the sensor. In one particular implementation, the microcontroller is configured for bi-directional communication with a remote processor over a single wire.

The system may be configured to receive and dynamically store a history of physiological parameters over a predetermined period of time, as well as a wide variety of other information, such as but not limited to: patient identification, patient information such as age, gender, weight, body mass index and/or other information, device identification such as serial number, model number, lot number and/or other information, calibration data or other data pertaining to the sensor itself, scaling factors, monitored physiological parameters over a predetermined period of time (e.g. over the past 8 hours, the past 24 hours, or other time period as desired), data about a cable or cables that connect the sensor system to the patient monitor, data about the patient monitor or monitors used in conjunction with the sensor, the date the sensor was manufactured, the shelf life of the sensor, the date and time when the sensor was first put into use and/or the length of time the sensor has been in use, the maximum time the sensor may be used, time since the patient monitor was first put into use, time since a patient cable was first put into use, location of the patient, signal quality indicators, fault or alarm codes, identification of doctor or other staff members, and/or other data that may be useful in a particular in a particular setting.

The invention further encompasses a method of sensing a biomedical parameter with a sensor having a local microprocessor and memory. The method may include, for example, transmitting a signal from a physiological sensor system over a cable. In one specific approach, a single wire protocol is employed to receive and transmit authentication data over a single wire between the sensor system and an external processor. The method also includes processing the sensor signal externally from the system at a remote processor, and then receiving data from the remote processor over a single wire of the cable and storing the data in memory.

The method may further include disconnecting the sensor system from a first external processor unit, such as a patient monitor or data box for example, at a first location. The sensor system, including the sensor and the microprocessor and memory that are local to the sensor, is transported to a second location, where the sensor system is connected to a second external processor unit. The sensor is authenticated at the second location. Data stored in the second memory is uploaded to the second external processor unit. The data may be any of a variety of different information, such as a history of physiological measurements over time, and/or other data.

Another aspect of the invention includes a method of sensing a biomedical parameter with a sensor system having a memory. Authentication information that is stored in the sensor memory is accessed by a remote processor to authenticate the sensor system. A sensor signal representative of a physiological parameter is transmitted from the sensor, and the sensor signal is processed at a remote processor. Data representative of a physiological parameter sensed by the sensor is then received from the remote processor and is stored in the sensor memory. In one approach, the data received from a remote processor and stored in the sensor memory includes a history of at least one physiological parameter of a patient over a predetermined period of time. The steps of accessing authentication information and receiving data from a remote processor may optionally utilize a single wire protocol.

Other objects, features and advantages of the invention will become apparent from a consideration of the following detailed description, the drawings, and the claims.

### BRIEF DESCRIPTION OF THE DRAWINGS

Having thus described aspects of the invention in general terms, reference will now be made to the accompanying drawings, which are not necessarily drawn to scale, and wherein:
Fig. 1 is a block diagram illustrating a sensor system having a microprocessor and memory local to the sensor, and a patient monitoring system in communication with the sensor system;
Fig. 2 is a schematic illustrating an embodiment in which a microprocessor local to the sensor communicates with a patient monitoring system over a single wire of a connector cable;
Fig. 3 is a perspective view of an embodiment of a sensor system having a microprocessor local to the sensor and a connector cable with which to communicate with an external processor;

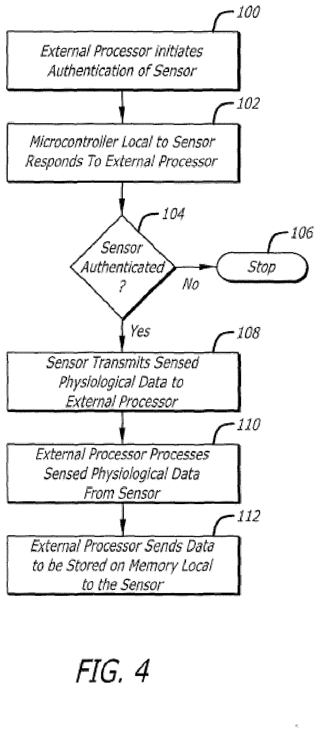

Fig. 4 is a flow diagram of authenticating a sensor, sending signals from the sensor to an external processor, and storing processed data on memory local to the sensor; and
Fig. 5 is a flow diagram illustrating the process of storing data on memory local to the sensor at a first location with a first external processor, then relocating the sensor and the memory local to the sensor to a second location and a second external processor.

### DETAILED DESCRIPTION OF A PREFERRED EMBODIMENT OF THE INVENTION

Aspects of the present invention now will be described more fully with reference to the accompanying drawings, in which some but not all embodiments of the invention are shown. Indeed, the invention may be embodied in many different forms and should not be construed as limited to the embodiments set forth herein; rather, these embodiments are provided so that this disclosure will satisfy applicable legal requirements. Like numbers refer to like elements throughout.

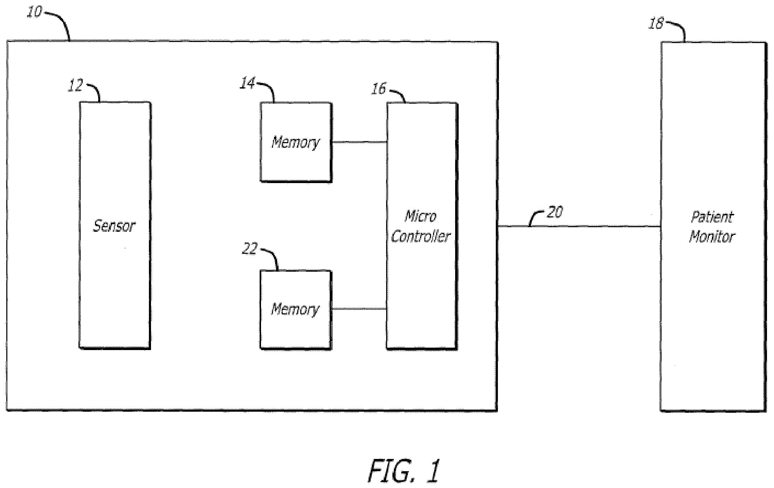

Generally, the present invention includes all, or portions of, a sensing system 10 (Fig. 1). Various embodiments or features of the invention will be presented in terms of systems that may include a number of devices, components, modules, and the like. It is to be understood and appreciated that the various systems may include additional devices, components, modules, etc. and/or may not include all of the devices, components, modules etc. discussed in connection with the figures. A combination of these approaches may also be used.

Various embodiments of the invention will also be presented herein using flow charts. It will be understood to one of ordinary skill in the art in view of this disclosure that some of the steps or actions described in a flow chart as taking place in a certain order may, in other embodiments, take place in a different order. Likewise, some of the steps or actions described in the flow charts may, in other embodiments, be performed simultaneously or combined into a single step or action.

As will be appreciated by one of skill in the art, the present invention may be embodied as a method (including, for example, a computer-implemented process), an apparatus (including, for example, a system, device, computer program product, etc.), or a combination of the foregoing. Accordingly, embodiments of the present invention may take the form an entirely hardware embodiment (e.g., an application-specific integrated circuit), or an embodiment combining software and hardware aspects that may generally be referred to herein as a "system." Furthermore, embodiments of the present invention may include a computer program product on a computer-readable medium having computer-executable program code embodied in the medium. As used herein, a processor may be "configured to" perform a certain function in a variety of ways, including, for example, by having one or more general-purpose circuits perform the function by executing particular computer-executable program code, and/or by having one or more application-specific circuits perform the function.

Computer-executable program code for carrying out operations of embodiments of the present invention may be written in an object-oriented, scripted or unscripted programming language such as Java, Perl, Smalltalk, C++, or the like. However, the computer-executable program code for carrying out operations of embodiments of the present invention may also be written in conventional procedural programming languages, such as the "C" programming language, the "BASIC" programming language or a variation thereof, or similar programming languages.

Embodiments of the present invention are described below with reference to flowchart illustrations and/or block diagrams of methods, apparatus, and computer program products. It will be understood that blocks of the flowchart illustrations and/or block diagrams, and/or combinations of blocks in the flowchart illustrations and/or block diagrams, can be implemented by computer program instructions embodied in computer-executable program code. These computer program instructions may be provided to a processor of a general purpose computer, special purpose computer, or other programmable data processing apparatus to produce a particular machine, such that the instructions, which execute via the processor of the computer or other programmable data processing apparatus, create means for implementing the functions/acts specified in the flowchart and/or block diagram block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function/act specified in the flowchart and/or block diagram block(s).

The computer program instructions may also be loaded onto a computer or other programmable data processing apparatus to cause a series of operational steps to be performed on the computer or other programmable apparatus to produce a computer-implemented process, such that the instructions which execute on the computer or other programmable apparatus provide steps for implementing the functions/acts specified in the flowchart and/or block diagram block(s). Alternatively, computer program implemented steps or acts may be combined with operator or human implemented steps or acts in order to carry out an embodiment of the invention.

In one embodiment of the present invention, and referring to Fig. 1, a physiological sensor system 10 includes a sensor 12 and associated monitoring system 18 are provided with a sensor integrity authentication system. The sensor system is provided with ROM 14, in which an encryption algorithm is stored. The sensor system is further provided with a microcontroller 16 to execute the encryption algorithm. The microcontroller 16 is in communication with a patient monitoring system 18, by means of a cable 20. When the monitoring system 18 executes handshaking software and sends a challenge to the sensor microcontroller 16, the sensor microcontroller 16 executes the encryption algorithm stored in memory 14 and responds appropriately to the monitoring system 18. The monitoring system verifies the response from the sensor and verifies it against pre-determined, acceptable responses, thereby authenticating the sensor 12 to the monitoring system 18. By returning a valid response, the sensor system 10 helps to ensure that the sensor 12 is appropriate for use with the monitoring system 18. On the other hand, if the sensor system does not return a valid response to the monitoring system, the monitoring system 18 may be configured to refuse to work with the sensor 12 or otherwise raise a flag to medical personnel that the integrity and quality of the sensor is not verified.

Fig. 1 is a simplified representation of a disposable sensor system 10 according to one embodiment of the invention. The sensor 12 may be a blood pressure transducer, a sensor to detect the flow and/or temperature of blood or another fluid, a blood glucose sensor, a hemoglobin sensor, a lactate sensor, various hemodynamic monitoring devices (measuring one or more of CO, SV, SVV, and SVR), oxygen saturation sensor (SvO₂, SpO₂, ScvO₂), or other sensor.

In Fig. 1, memories 14 and 22 and a microcontroller 16 are fixedly attached to the sensor 12. The microcontroller is in communication with patient monitoring instruments 18, which include at least one processor. It should be understood that, as used herein, the terms "monitor," "patient monitor," and "patient monitoring instruments" may refer to a variety of configurations. For example, in some patient monitoring systems, a primary processor and memory are housed together with a display. In others, the primary processor and memory is housed in a "data box" that is separate from the display. Consequently, the monitoring systems discussed herein encompass a variety of systems that interface with one or more sensors to monitor at least one physiological parameter of a patient. Most such monitoring systems include a display, and may be part of a monitoring network.

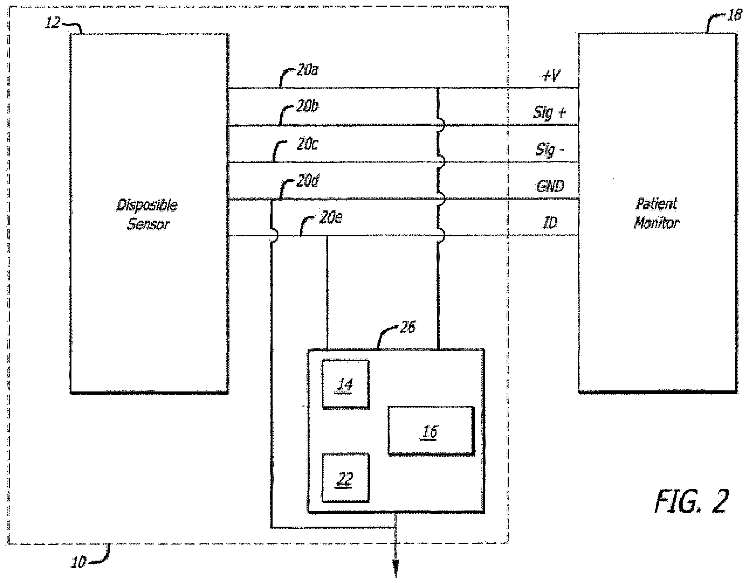

In one embodiment, the disposable unit 10 connects to the patient monitoring instrument 18 with a five wire cable 20. Referring to Fig. 2, four of the wires provide excitation voltage (20a), positive and negative signals from the sensor (20b and c), and a ground wire (20d), respectively. The microcontroller 16 communicates with the patient monitoring system 18 over the fifth wire (20e), using a single wire protocol to transmit and receive data over the single wire 20e. The single wire protocol offers an efficient and elegant means for the sensor microcontroller to communicate with the monitoring system.

In other embodiments, the cable may have more or fewer than five wires, depending on the configuration of the monitoring system. For instance, in an alternative embodiment, two wires may be provided for communication between the microcontroller and the monitoring system. In other embodiments, the cable (*e.g.* cable 20 in Fig. 1) may include passive and/or active components such as, for example, resistors, capacitors, multi-wire circuitry, and/or other electronic components that work together with the sensor and/or the patient monitor.

Memory 14, 22 that is local to the sensor is also provided (Fig. 1). A first ROM 14 has 128 bytes of memory and 64 bytes for an encryption algorithm. The memory is a single wire interface memory such as, for example, the Maxim DS28E01 sold by Maxim of Sunnyvale, California. In this specific embodiment, 1024 bits of EEPROM is combined with challenge-and-response authentication security implemented with the ISO/IEC 10118-3 Secure Hash Algorithm (SHA-1). The 1024-bit EEPROM array is configured as four pages of 256 bits with a 64-bit scratchpad to perform write operations. This memory communicates over a single-contact single-wire bus. The registration number is burned into memory, and may act as the node address in the case of a multi-device single wire network. Other memories suitable for storage of an authentication algorithm may alternatively be utilized.

Additional memory 22 is provided in which to store data that is useful to transport with the disposable sensor. For example, in a typical cardiac procedure in a hospital, the patient moves from pre-op to the operating room to the Intensive Care Unit and so on. At each location, there may be a separate patient monitor. The disposable device 10 may be disconnected from a first monitor at a first location, and then moved with the patient to a second location at which there is a second, different monitor. Data stored in the memory 22 on the disposable device may then be uploaded to the new monitor.

In one embodiment, this additional memory 22 is provided as an EEPROM. In one embodiment, the EEPROM may be a Microchip Technology Inc. EEPROM, such as the EEPROM 11AA160, which is a family of 16 Kbit serial electrically erasable PROMs. The devices are organized in blocks of x8-bit memory and support the single I/O UNI/O serial bus. By using Manchester encoding techniques, the clock and data are combined into a single, serial bit stream (SCIO), where the clock signal is extracted by the receiver to correctly decode the timing and value of each bit.

The microcontroller serves, among other things, to communicate with the external monitoring system, to execute the authentication function, and to write information to be stored onto the ROM. Non-limiting specific examples of particular suitable microcontrollers are the PIC12F508/509/16F505 devices from Microchip Technology and the Atmel ATmega168P-10MU. These are highly-integrated, low cost, high performance, 8-bit, fully static, Flash-based CMOS microcontrollers, for their intended use as part of a disposable sensor. The microcontroller employs a RISC architecture with only 33 single-word/single-cycle instructions. All instructions are single cycle (200 µs) except for program branches, which take two cycles. The 12-bit wide instructions are highly symmetrical. The easy-to-use and easy to remember instruction set reduces development time significantly, as compared to microcontrollers using more complicated instruction sets. This type of microcontroller has sufficient processor speed, memory capacity, and cost efficiency, and has a sufficiently small footprint for use in conjunction with sensors of physiological sensors.

The PIC12F508/509/16F505 products are equipped with special features that reduce system cost and power requirements. The Power-on Reset (POR) and Device Reset Timer (DRT) eliminate the need for external Reset circuitry. There are four oscillator configurations to choose from (six on the PIC16F505), including INTRC Internal Oscillator mode and the power-saving, LP (Low-Power) Oscillator mode. Power-Saving Sleep mode, Watchdog Timer and code protection features improve system cost, power and reliability.

Examples of data that may be stored in sensor memory 22 include patient identification, patient information such as age, gender, weight, body mass index and/or other information, device identification such as serial number, model number, lot number and/or other information, calibration data or other data pertaining to the sensor itself, monitored physiological parameters over a predetermined period of time (e.g. over the past 8 hours, the past 24 hours, or other time period as desired), data about the cable or cables that connect the sensor to the patient monitor such as serial number and/or lot, data about the patient monitor or monitors used with the sensor, the date the sensor was manufactured, the shelf life of the sensor, the date and time when the sensor was first put into use and/or the length of time the sensor has been in use, the maximum time the sensor may be used, time since the patient monitor was first put into use, time since a patient cable was first put into use, location of the patient, signal quality indicators, fault or alarm codes, name of doctor or other staff members, and/or other data that may be useful in a particular embodiment.

Considering now the processing of signals from the sensor 12, in a presently preferred embodiment, data from the sensor 12 is communicated over cable 20 to the patient monitor 18, at which one or more processors process the raw signals from the sensor. The patient monitor 18 then sends all or selected portions of the processed data over wire 20e (Fig. 2) to the microcontroller 16, which stores the data in the sensor memory 22. For example, a pressure sensor may output raw signals to the patient monitor 18, which then calculates cardiac pressure values. These cardiac pressure values and/or other parameters are periodically sent from the patient monitor 18 over the cable 20 and are stored in memory 22.

In some embodiments, it may be desirable to store raw signals from the sensor 12 in the sensor memory 22. In most embodiments, the raw signals from the sensor 12 would first be sent to the patient monitor 18, which would then retransmit the raw sensor signals or selected raw sensor signals to the microcontroller 16 for storage in the sensor memory 22. In this alternative embodiment, the sensor 12 would be equipped with a significant amount of memory to accommodate hours and/or days of raw sensor data and/or processed algorithm data. In one such embodiment in which raw sensor data is stored, the sensor includes at least 2 gigabytes and preferably 8 gigabytes of memory on which to store raw sensor data.

Fault codes may also be stored in the memory 22 so that, for example, if the monitoring system 18 or sensor 12 or cable 20 or other component fails at some point, a time and/or date stamped record of the fault codes at about the time of the failure may later be accessed for analysis purposes. Other information, such as for example patient height, weight, or BMI, how many and what type of catheters have been used on the particular patient monitor, information about the monitor itself, the version of software the monitor is running, information about the hospital that the monitor is in, and/or other information that might be used for later analysis and business decision-making may also be stored.

Some information stored in the sensor memory, such as sensor calibration data, manufacturing lot number, serial number, time and/or date of production, may be written onto memory at the manufacturing facility. Other information may be stored by the patient monitor or other means at a treatment site. Considering calibration information in particular, sensor performance measurements may be written to the memory during the manufacturing/testing process. Consequently, if there is an offset of for example 2 mm of mercury for a particular sensor, that information may be stored in memory on the sensor at the manufacturing facility. In use, the patient monitor 18 may use the calibration information stored in the sensor memory to make more accurate calculations from signals received from the sensor 12. The authentication function may be used to ensure that the sensor is of a type that has such calibration information stored thereon.

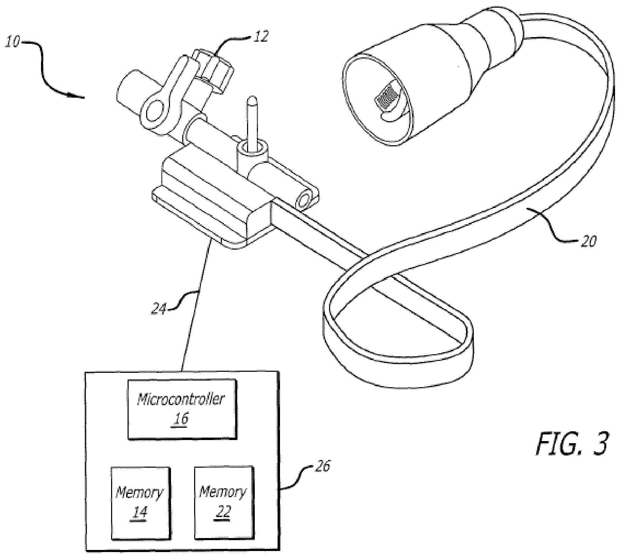

Considering the embodiment of Fig. 3, the disposable sensor unit 10 includes a sensor 12 that is connected by a cable 24 to the microcontroller 16 and local memory 14, 22 in a unit 26. Thus, the microcontroller 16 and memory 14, 22 are local to the sensor 12, but spaced a distance away. The cable 24 may be unattachable, such that the sensor 12 and the microcontroller/memory unit 26 are fixedly attached. This arrangement is advantageous in a number of situations such as when, for example, attaching a microcontroller and memory directly to the sensor would interfere with the operation of the sensor, or where there is not room onboard the sensor for the microcontroller and memory.

In an alternative embodiment, the patient monitoring system acts as a hub. It gathers certain information from a sensor or sensors, makes appropriate calculations, displays information and/or performs other functions. Other information may be input into the patient monitoring system manually, such as by a nurse or other medical personnel, and/or collected from other sources. The monitoring system then sends selected information that it has gathered, calculated or otherwise obtained to memory connected to the sensor for storage on the sensor. This information stored on the sensor memory is then portable with the sensor even when the sensor is disconnected from the monitoring system. The patient may move from room-to-room or place-to-place and, when the patient arrives at a new room or place, the sensor may be connected to another monitoring system that then uploads data stored on the sensor memory. In instances where the sensor is sent to a manufacturing facility, a lab, or the like, the information stored on the sensor may be accessed for analysis.

While in a preferred embodiment of the present invention the sensor 10 is provided with a microprocessor, in an alternative embodiment, a single wire interface EEPROM may interface directly with a monitoring system, without a microcontroller. Non-limiting examples of presently suitable single wire interface EEPROMS for this alternative embodiment include the Maxim DS28E01 and the Microchip 11AA160 EEPROM. This approach is advantageous over EEPROMs that require additional wires in the cable, a greater number of pins and/or significant power requirements.

A further alternative embodiment is to utilize a microprocessor having embedded cryptographic capability, such that it is not necessary to store an authentication algorithm in ROM separately from the microcontroller. Suitable microcontrollers for this purpose are available from Atmel Corporation of San Jose, California, among others.

In embodiments in which the sensor is a blood glucose sensor, specific information that may be stored in memory that is local to the sensor may include information of the blood access device to which the sensor is connected, such as manufacturer, size, length, model number, lumen and lumen volume and information on the type of flow profile that is needed, as well as other information specifically helpful to the functioning of a blood glucose sensor. Also, a check in the manufacturing process may be instituted in which a standard sensor test is made during the manufacturing process. The values sensed by the sensor are recorded and stored on the sensor memory. In the field, an optional test may be made in which the values sensed by the sensor are compared with the values stored in the sensor memory from the earlier test. If the values are significantly different, it may indicate that the sensor is damaged and should no longer be used. The patient monitor, for example, may decline to use a sensor that is possibly damaged, or may raise a flag to medical personnel to indicate a possible problem with the sensor.

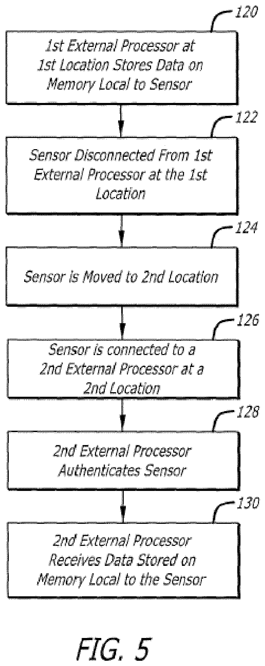

Fig. 5 illustrates one embodiment of a method for sensing a physiological parameter and storing information locally to the sensor. In step 100, the external processor in the patient monitoring unit initiates authentication of the sensor by transmitting to the microcontroller that is local to the sensor. In a presently preferred embodiment, this transmission is made over a cable connecting the sensor unit to the patient monitoring unit, as illustrated in Fig. 2. When a single wire protocol is used, the communication between the patient monitoring unit and the microcontroller is done over a single wire on the cable. In step 102, the microcontroller local to the sensor implements the authentication algorithm, and responds to the external processor. At step 104, upon receiving a valid response or responses from the microcontroller, the patient monitoring unit deems the sensor to be valid.

On the other hand, if the sensor does not issue the proper response, the sensor is not validated. For an invalid sensor, the patient monitoring unit may be configured to respond in any of a variety of different ways. The monitoring unit may simply refuse to accept signals from the sensor, make no calculations of data based on the signals, and generally decline to undertake the monitoring function, as at step 106 in Fig. 5. Alternatively, the monitoring unit may be configured to indeed process signals received from the sensor, but to display a warning message or otherwise flag the information as possibly unreliable.

At step 108, the sensor transmits sensed signals to the external processor which, at step 110, processes the signals from the sensor. At step 112, the external processor transmits data to be stored on the memory that is local to the sensor. In one embodiment, the data is transmitted over a cable and, in a further embodiment, over a single wire of the cable using a single wire protocol.

Fig. 6 illustrates a method for storing data on memory local to the sensor, so that the data will travel with the sensor as a patient is moved from location-to-location, and from monitor-to-monitor. When a sensor is disconnected from one monitor and connected to another, certain data must be input again into the second monitor, often manually. Also, any history of physiological data stored on the first monitor remains on the first monitor and is not available at the second monitor.

In Fig. 6, though, a first external processor at a first location stores data on memory that is local to the sensor, at step 120. In a presently preferred embodiment, this first external processor is part of a first patient monitoring device. At step 122, the sensor is disconnected from the first external processor at the first location. The sensor is then moved, in step, 124 to a second location, such as would be the case when the patient is moved from post-op to another location. The sensor is then connected to a second external processor at the second location, the second external processor being part of another patient monitoring device, at step 126. Optionally, the second external processor authenticates the sensor, at step 128, and then receives data that has been stored on the memory that is local to the sensor. In this way, a second patient monitoring device may gather historical physiological data about the patient as developed earlier at the first patient monitoring device. By storing such information on the memory that is local to the sensor, the patient may be moved from monitor-to-monitor-to-monitor without the loss of data, since data is stored on the memory local to the sensor.

While certain exemplary embodiments have been described and shown in the accompanying drawings, it is to be understood that such embodiments are merely illustrative of and not restrictive on the broad invention, and that this invention not be limited to the specific constructions and arrangements shown and described, since various other changes, combinations, omissions, modifications and substitutions, in addition to those set forth in the above paragraphs, are possible. Those skilled in the art will appreciate that various adaptations and modifications of the just described embodiments can be configured without departing from the scope of the invention, and that particular embodiments of the invention may have additional advantages, such as EMI noise reduction and/or providing an environment for using multiple instruments simultaneously. Therefore, it is to be understood that, within the scope of the appended claims, the invention may be practiced other than as specifically described herein.

## Claims

1. A system (10) for sensing a physiological parameter for use in conjunction with
a remote processor that is external to the system, the system comprising:
a physiological sensor (12) coupled to the remote processor, and adapted to output a sensor signal representative of a sensed physiological parameter to the remote processor;
a microcontroller (16) located locally within the system and fixedly attached to the physiological sensor (12), the microcontroller adapted to exchange data with the remote processor, the microcontroller (16) having a first memory (14) and a second memory (22); wherein the first memory (14) is adapted to store an authentication algorithm and the microcontroller (16) configured to engage in an authentication process through the authentication algorithm to ensure that the physiological sensor is appropriate for use when queried by the remote processor; **characterised in that** the second memory (22) is configured to receive processed sensor data from the remote processor and store at least the processed sensor data and calibration information pertaining to the physiological sensor (12); and
wherein the authentication process is configured to ensure that the physiological sensor is of a type that has calibration information stored thereon.

2. The system according to claim 1,
wherein the first memory (14) comprises a single wire interface memory.

3. The system according to claim 1 or 2,
wherein the sensor (12) comprises one of a pulse oximetry sensor, a blood glucose sensor, and a pressure transmitter.

4. The system according to one of the preceding claims,
wherein the authentication algorithm comprises a challenge-and-response hash algorithm.

5. The system according to one of the preceding claims,
wherein the microcontroller (16) is fixedly attached to the sensor (12) by a cable.

6. The system according to claim 5,
wherein the cable includes further electronic components.

7. The system according to one of the preceding claims,
wherein the microcontroller (16) and sensor (12) are housed together.

8. The system according to one of the preceding claims,
wherein the second memory (22) is configured to receive and store data concerning at least one of faults, device ID, sensor calibration, date sensor first put into use, accumulated time of use of the sensor, number of times sensor is plugged and unplugged from a patient monitor, temperature, patient information, device identification information, one or more scaling factors, monitored physiological parameters over a predetermined period of time, information about equipment used in conjunction with the sensor, manufacture date of the sensor, shelf life of the sensor, patient identifying information, patient height, patient weight, patient BMI, patient location, signal quality, and medical personnel utilizing the device.

9. The system according to one of the preceding claims,
wherein the second memory (22) is configured to store a history of biomedical data of a patient over a predetermined period of time.

10. The system according to one of the preceding claims,
wherein the capacity of the second memory (22) is selected so that both raw sensor data and processed sensor data may be stored thereon.

11. The system according to one of the preceding claims,
wherein the second memory (22) is configured to receive and store raw sensor data.

12. A method of sensing a biomedical parameter with a physiological sensor (12) having a microcontroller (16) and memory (14, 22) located locally within the system and fixedly attached to the physiological sensor (12), the method utilising a system (10) as defined in claim 1 and comprising the steps of:
exchanging, by the microcontroller (16) and through an authentication algorithm stored in the memory (14), authentication data for an authentication process with a remote processor external to the system to ensure that the physiological sensor (12) is appropriate for use;
transmitting, by the microcontroller, a sensor signal representative of a sensed biomedical parameter from the physiological sensor;
receiving, by the microcontroller, processed sensor data from the remote processor and storing at least the processed sensor data and calibration information pertaining to the physiological sensor in the memory (22); and
wherein the authentication process is configured to ensure that the physiological sensor is of a type that has calibration information stored thereon.

13. The method according to claim 12, further comprising the steps of:
disconnecting the system as defined in claim 1 from a first external processor unit at a first location;
transporting the system to a second location;
connecting the system to a second external processor unit at the second location;
authenticating the sensor at the second location; and
uploading data stored in the second memory to the second external processor unit.

14. The method according to on of the claims 12 to 13,
wherein storing data in the sensor memory comprises storing a history of at least one physiological parameter of a patient over a predetermined period of time.

15. The method according to on of the claims 12 to 14,
wherein the steps of exchanging authentication information and receiving data from a remote processor comprise utilizing a single wire protocol.

## Patentansprüche

1. System (10) zum Erfassen eines physiologischen Parameters zur Verwendung in Verbindung mit einem fernen Prozessor, der außerhalb des Systems liegt, das System umfassend:
einen physiologischen Sensor (12),
gekoppelt mit dem fernen Prozessor und ausgelegt, ein Sensorsignal an den fernen Prozessor auszugeben, das repräsentativ für einen erfassten physiologischen Parameter ist;
einen Mikrocontroller (16),
befindlich örtlich innerhalb des Systems und fest an dem physiologischen Sensor (12) angebracht, wobei der Mikrocontroller ausgelegt ist, Daten mit dem fernen Prozessor auszutauschen, wobei der Mikrocontroller (16) einen ersten Speicher (14) und einen zweiten Speicher (22) aufweist;
wobei der erste Speicher (14)
ausgelegt ist, einen Authentifizierungsalgorithmus zu speichern, und der Mikrocontroller (16) ausgelegt ist, an einem Authentifizierungsvorgang durch den Authentifizierungsalgorithmus teilzunehmen, um sicherzustellen, dass der physiologische Sensor zur Verwendung geeignet ist, wenn er durch den fernen Prozessor abgefragt wird;
**dadurch gekennzeichnet, dass** der zweite Speicher (22) ausgelegt ist, bearbeitete Sensordaten von dem fernen Prozessor zu empfangen und zumindest die bearbeiteten Sensordaten und Kalibrierungsinformationen bezüglich des physiologischen Sensors (12) zu speichern; und
wobei der Authentifizierungsvorgang ausgelegt ist sicherzustellen, dass der physiologische Sensor von einem Typ ist, der darin Kalibrierungsinformationen gespeichert hat.

2. System nach Anspruch 1,
wobei der erste Speicher (14) einen Eindrahtschnittstellenspeicher umfasst.

3. System nach Anspruch 1 oder 2,
wobei der Sensor (12) einen aus einem Pulsoxymetriesensor, einem Blutglucosesensor und einem Druckaufnehmer umfasst.

4. System nach einem der vorangegangenen Ansprüche,
wobei der Authentifizierungsalgorithmus einen Challenge-Response-Hash-Algorithmus umfasst.

5. System nach einem der vorangegangenen Ansprüche,
wobei der Mikrocontroller (16) über ein Kabel fest am Sensor (12) angebracht ist.

6. System nach Anspruch 5,
wobei das Kabel weitere elektronische Bauteile enthält.

7. System nach einem der vorangegangenen Ansprüche,
wobei der Mikrocontroller (16) und der Sensor (12) zusammen in einem Gehäuse untergebracht sind.

8. System nach einem der vorangegangenen Ansprüche,
wobei der zweite Speicher (22) ausgelegt ist, Daten zu empfangen und zu speichern, betreffend mindestens eines aus Störungen, Geräte-ID, Sensorkalibrierung, Erstverwendungsdatum des Sensors, akkumulierter Nutzungsdauer des Sensors, Anzahl der Male, die der Sensor an einen Patientenmonitor gesteckt und davon abgenommen wurde, Temperatur, Patienteninformationen, Geräte-Identifikationsinformationen, einem oder mehrere Skalierungsfaktoren, überwachten physiologischen Parametern über einen vorgegebenen Zeitraum, Informationen über in Verbindung mit dem Sensor benutzte Einrichtungen, Herstellungsdatum des Sensors, Haltbarkeitsdauer des Sensors, Patienten-Identifikationsinformationen, Patientenhöhe, Patientengewicht, Patienten-BMI, Patientenort, Signalqualität und medizinischem Personal, das die Vorrichtung verwendet.

9. System nach einem der vorangegangenen Ansprüche,
wobei der zweite Speicher (22) ausgelegt ist, einen Verlauf biomedizinischer Daten eines Patienten über einen vorgegebenen Zeitraum zu speichern.

10. System nach einem der vorangegangenen Ansprüche,
wobei die Kapazität des zweiten Speichers (22) so gewählt ist, dass sowohl Sensorrohdaten als auch bearbeitete Sensordaten darin gespeichert werden können.

11. System nach einem der vorangegangenen Ansprüche,
wobei der zweite Speicher (22) ausgelegt ist, Sensorrohdaten zu empfangen und zu speichern.

12. Verfahren des Erfassens eines biomedizinischen Parameters mit einem physiologischen Sensor (12) mit einem Mikrocontroller (16) und Speicher (14, 22), befindlich örtlich in dem System und fest an dem physiologischen Sensor (12) angebracht, wobei das Verfahren ein System (10) verwendet, wie es in Anspruch 1 beschrieben ist, und folgende Schritte umfasst:
Austauschen, über den Mikrocontroller (16) und mittels eines in dem Speicher (14) gespeicherten Authentifizierungsalgorithmus, von Authentifizierungsdaten für einen Authentifizierungsvorgang mit einem fernen Prozessor außerhalb des Systems, um sicherzustellen, dass der physiologische Sensor (12) zur Verwendung geeignet ist;
Senden, über den Mikrocontroller, eines Sensorsignals, das repräsentativ für einen erfassten physiologischen Parameter von dem physiologischen Sensor ist;
Empfangen, durch den Mikrocontroller, von bearbeiteten Sensordaten von dem fernen Prozessor und Speichern zumindest der bearbeiteten Sensordaten und Kalibrierungsinformationen bezüglich des physiologischen Sensors in dem Speicher (22);
und
wobei der Authentifizierungsvorgang ausgelegt ist sicherzustellen, dass der physiologische Sensor von einem Typ ist, der darin Kalibrierungsinformationen gespeichert hat.

13. Verfahren nach Anspruch 12, weiter umfassend die folgenden Schritte:
Trennen des Systems wie in Anspruch 1 beschrieben von einer ersten externen Prozessoreinheit an einem ersten Ort;
Transportieren des Systems zu einem zweiten Ort;
Verbinden des Systems mit einer zweiten externen Prozessoreinheit an dem zweiten Ort;
Authentifizieren des Sensors an dem zweiten Ort; und
Hochladen von in dem zweiten Speicher gespeicherten Daten in die zweite externe Prozessoreinheit.

14. Verfahren nach einem der Ansprüche 12 bis 13,
wobei das Speichern von Daten im Sensorspeicher ein Speichern eines Verlaufs mindestens eines physiologischen Parameters eines Patienten über einen vorgegebenen Zeitraum umfasst.

15. Verfahren nach einem der Ansprüche 12 bis 14,
wobei die Schritte des Austauschens von Authentifizierungsinformationen und Empfangens von Daten von einem fernen Prozessor die Verwendung eines Eindrahtprotokolls umfasst.

## Revendications

1. Système (10) de détection d'un paramètre physiologique à utiliser en conjonction avec
un processeur éloigné, qui est extérieur au système, le système comprenant;
un capteur (12) physiologique
couplé au processeur éloigné et conçu
pour envoyer au processeur éloigné un signal de capteur représentatif du paramètre physiologique détecté;
une unité (16) de microcommande
disposée localement dans le système et
fixée au capteur (12) physiologique,
l'unité de microcommande étant conçue pour
échanger des données avec le processeur éloigné, l'unité (16) de microcommande ayant une première
mémoire (14) et une deuxième mémoire (22);
dans lequel la première mémoire (14)
est conçue pour mémoriser un algorithme d'authentification et l'unité (16) de microcommande
est configurée pour s'engager dans une opération d'authentification par l'intermédiaire de l'algorithme d'authentification, afin de s'assurer que le capteur physiologique est approprié à l'utilisation sur demande du processeur éloigné;
**caractérisé en ce que** la deuxième mémoire (22)
est configurée pour recevoir des données traitées
de capteur du processeur éloigné et pour mémoriser au moins les données traitées de capteur et une information d'étalonnage concernant le capteur (12) physiologique et
dans lequel l'opération d'authentification est configurée pour s'assurer que le capteur physiologique est du type sur lequel est mémorisé une information d'étalonnage.

2. Système suivant la revendication 1,
dans lequel la première mémoire (14)
comprend une mémoire à interface monofilaire.

3. Système suivant la revendication 1 ou 2,
dans lequel le capteur (12)
comprend un capteur d'oxymétrie à impulsion, un capteur du glucose dans le sang et un transmetteur de pression.

4. Système suivant l'une des revendications précédentes,
dans lequel l'algorithme d'authentification comprend un algorithme hash à challenge et réponse.

5. Système suivant l'une des revendications précédentes,
dans lequel l'unité (16) de microcommande est fixée au capteur (12) par un câble.

6. Système suivant la revendication 5,
dans lequel le câble comprend d'autres composants électroniques.

7. Système suivant l'une des revendications précédentes,
dans lequel l'unité (16) de microcommande et le capteur (12) sont logés ensemble.

8. Système suivant l'une des revendications précédentes,
dans lequel la deuxième mémoire (22) est configurée pour recevoir et mémoriser des données concernant au moins l'un de défaut de dispositif ID, d'étalonnage de capteur, de capteur de date de première mise en utilisation, de temps cumulé d'utilisation du capteur, de nombre de fois où le capteur est enfiché et désenfiché d'un moniteur de patient, de température, d'information de patient, de température, d'information d'identification de dispositif, d'un ou plusieurs facteurs de mise à l'échelle, de paramètres physiologiques surveillés sur un laps de temps déterminé à l'avance, d'information sur un équipement utilisé en conjonction avec le capteur, de date de fabrication du capteur, de durée de vie du capteur, d'information d'identification du patient, de taille du patient, de poids du patient, d'indice de masse corporelle du patient, d'emplacement du patient, de qualité du signal et de personnel médical utilisant le dispositif.

9. Système suivant l'une des revendications précédentes,
dans lequel la deuxième mémoire (22)
est configurée pour mémoriser un historique de données biomédicales d'un patient sur un laps de temps déterminé à l'avance.

10. Système suivant l'une des revendications précédentes,
dans lequel la capacité de deuxième mémoire (22) est choisie de manière à pouvoir y mémoriser à la fois des données brutes de capteur et des données traitées de capteur.

11. Système suivant l'une des revendications précédentes,
dans lequel la deuxième mémoire (22)
est configurée pour recevoir et mémoriser des données brutes de capteur.

12. Procédé de détection d'un paramètre biomédical par un capteur (12) physiologique ayant une unité (16) de microcommande et une mémoire (14, 22) placées localement dans le système et
fixées au capteur (12) physiologique, le procédé utilisant un système (10) tel que défini à la revendication 1 et comprenant les stades de:
échange, par l'unité (16) de microcommande
et par l'intermédiaire d'un algorithme d'authentification mémorisé dans la mémoire (14)
de données d'identification pour une opération d'identification avec un processeur éloigné extérieur au système pour s'assurer que le capteur (12) physiologique est approprié à être utilisé ;
émission, par l'unité de microcommande, d'un signal de capteur représentatif d'un paramètre biomédical détecté par le capteur physiologique;
réception, par l'unité de microcommande, de données de capteur traitées à partir du processeur éloigné et mémorisation au moins des données de capteur traitées et d'une information d'étalonnage concernant le capteur physiologique dans la mémoire (22) et
dans lequel l'opération d'identification est configurée pour s'assurer que le capteur physiologique est d'un type qui a une information d'étalonnage qui y est mémorisée.

13. Procédé suivant la revendication 12, comprenant en outre les stades de:
déconnexion du système tel que défini à la revendication 1 d'une première unité extérieure de processeur en un premier emplacement;
transport du système à un deuxième emplacement;
connexion du système à une deuxième unité de processeur extérieur au deuxième emplacement;
authentification du capteur au deuxième emplacement et
charge de données mémorisées dans la deuxième mémoire dans la deuxième unité de processeur extérieur.

14. Procédé suivant l'une des revendications 12 à 13,
dans lequel, mémoriser des données dans la mémoire de capteur, comprend mémoriser un historique d'au moins un paramètre physiologique d'un patient sur un laps de temps déterminé à l'avance.

15. Procédé suivant l'une des revendications 12 à 14,
dans lequel les stades d'échange d'authentification d'information et de réception de données à partir d'un processeur éloigné comprennent utiliser un protocole monofilaire.
